Europäisches Patentamt

European Patent Office

Office européen des brevets

(11) Publication number: **0 437 789 A2**

# EUROPEAN PATENT APPLICATION

(21) Application number: **90124889.8**

(22) Date of filing: **20.12.90**

(51) Int. Cl.⁵: **G01N 33/487**, C12Q 1/58, C12Q 1/34, C12Q 1/00

(30) Priority: **19.01.90 SE 9000192**

(43) Date of publication of application: **24.07.91 Bulletin 91/30**

(84) Designated Contracting States: **BE CH DE DK ES FR GB IT LI NL**

(71) Applicant: **GAMBRO AB** Post Box 10101 **S-220 10 Lund(SE)**

(72) Inventor: **Hakansson, Hakan** **Astrakanvägen 6** **S-223 56 Lund(SE)** Inventor: **Mattiasson, Bo** **Trädgardsmästaren 26** **S-222 48 Lund(SE)**

(74) Representative: **Boberg, Nils Gunnar Erik** **Gambro AB Patent Department Box 10101** **S-220 10 Lund(SE)**

(54) **A system, respectively a method, for measuring the concentration of a substance in a solution or dispersion thereof, whereby the substance consists of a compound which is non-electrically conducting or very slightly conducting.**

(57) The present invention relates to a system, respectively a method for measuring the concentration of a substance in a solution or dispersion thereof, whereby the substance consists of a compound which is non-electrically conducting or very slightly conducting.

The invention is primarily intended to be used for measuring the concentration of compounds or substances of medical interest, for example urea concentration in blood of a patient during for example hemodialysis, hemofiltration or hemodiafiltration. The conversion of the compound hereby occurs with a help of urease.

*Fig. 1*

EP 0 437 789 A2

## A SYSTEM, RESPECTIVELY A METHOD, FOR MEASURING THE CONCENTRATION OF A SUBSTANCE IN A SOLUTION OR DISPERSION THEREOF, WHEREBY THE SUBSTANCE CONSISTS OF A COMPOUND WHICH IS NON-ELECTRICALLY CONDUCTING OR VERY SLIGHTLY CONDUCTING

TECHNICAL FIELD

The present invention relates to a system, respectively a method, for measuring the concentration of a substance in a solution or dispersion thereof, whereby the substance consists of a compound which is non-electrically conducting or very slightly conducting.

The measuring of the concentration is primarily intended for compounds or substances of medical interest, for example the urea concentration in the patients blood during for example hemodialysis, hemofiltration, or hemodiafiltration.

BACKGROUND ART

The system according to the invention is based to a certain extent on previously known systems, which are described by way of example in American Patents Nos. 4 229 542, 4 311 789, 4 123 353, 4 266 021 and 4 587 219. The contents of these patents are hereby included in the present description. The contents of the European Patent Application No. EP 323 562 are also included in the present description.

With the help of the above-mentioned known system the concentration of a low molecular weight substance in a complex solution can be measured. This hereby occurs in that the substance is dialyzed out of the complex solution and is converted with the help of an enzyme column or similar during formation of or consumption of a substance, whose concentration is measured. The measuring can hereby advantageously occur in an electrically conducting fluid, for example a physiologically acceptable solution of sodium chloride in water and this can occur with a help of, for example, an $O_2$-electrode or a pH-meter. The known systems are, however, not suitable for all compounds since they are limited to the specificness of oxygen, respectively pH-sensors.

DISCLOSURE OF THE INVENTION

The present invention has come about in order to solve the above-mentioned problem. The system according to the invention can, however, be used more generally.

According to the invention there is thus achieved a system, respectively a method, for measuring the concentration of a substance in a solution or dispersion thereof, whereby the substance consists of a compound which is non-electrically conducting or very slightly conducting.

The system according to the invention is characterized by means for converting the substance in such a way that electrically conducting ions appear, and by means, for measuring these ions which are proportional to said degree of concentration. The method according to the invention is characterized instead in that the compound is converted in such a way that electrically conducting ions appear and that these ions are measured, the ions being proportional to the degree of concentration of the substance.

Preferably said means for converting the substance consists of an enzyme column in the same way as in the above-mentioned systems. The conversion can however also occur in other ways, for example through the addition of suitable chemically active compounds.

Preferably said means for measuring the appearing ions consists of a conductivity meter. Such meters have, in practice, shown themselves to be particularly reliable.

Should said substance occur in an electrically conducting solution, then the system according to the invention can include means for transferring the substance in its original form, or after converting appearing ions, to an electrically non-conducting or very slightly conducting solution before the measuring of the ions. In this way particularly low concentrations of the substance become more easily measurable.

Said means for transferring the substance or after converting appearing ions can consists of a dialyzer, a filter or similar, with the help of which the transferring can occur by diffusion and/or filtration.

According to a preferred application of the method according to the invention, the above-mentioned conversion appropriately occurs with a help of an enzyme column, whilst at the same time the measuring of appearing ions appropriately occurs with a help of a conductivity meter. This can occur substantially in the way described in the above-mentioned American Patents. It is only the measuring instrument itself which needs to be exchanged for a conductivity meter, whilst at the same time a suitable dialysis fluid is chosen, that is to say if dialysis is necessary.

Should the sought after substance occur in an electrically conducting solution, then it is first transferred to an electrically non-conducting or very slightly conducting solution before the measuring occurs. Preferably both conversion and measuring hereby occur after the transferring. This is to avoid interference from other compounds in the solution.

The measuring can advantageously be carried out in a water solution which is prepared with for example distilled water.

Preferably the measuring is carried out in a solution of low or non-conducting aminoacides for example L and/or D-alanine, L and/or D-histidine, L and/or D-serine or glycine, preferably glycine.

As mentioned above the method according to the invention is primarily intended to be used for measuring urea, for example in blood, whereby the conversion preferably occurs with the help of the enzyme urease. The methods can however also be used by measuring other compounds which are interesting particularly from a medical view of points. Preferably an enzyme is hereby used which is active on carbon-nitrogen bonds for measuring the present substance in anyone of the following combinations;

Asparaginase - L-Asparagine

Glutaminase - L-Glutamine

Penicillin amidase - Penicillin

Aminoacylase - N-acyl-amino acid

Citrullinase - L-Citrulline

Glutamin-(asparagin-)ase - L-Glutamine

preferably Urease - Urea.


BRIEF DESCRIPTION OF THE DRAWINGS

Fig 1 shows a simple block diagram of a system according to the invention.

Fig 2 shows the result of a continuous measuring of urea in a patient during hemodialysis.

Fig 3 shows how the concentration of sodium chloride can affect the measured results during measuring of urea in a water solution of glycine.

Fig 4 shows how glycine can be exchanged for certain other compounds without undue influence on the measured results. The various compounds are also compared with a 0.9% water solution of sodium chloride.


BEST MODE OF CARRYING OUT THE INVENTION

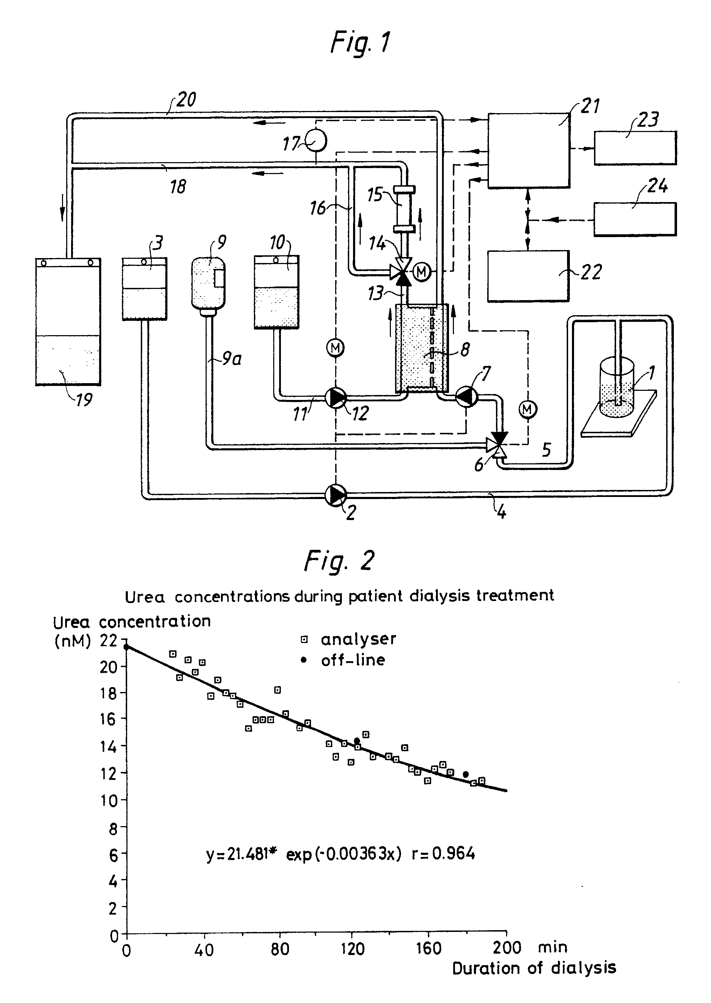

Fig 1 hereby shows a simple block diagram for a system according to the invention. According to this system a sample can be taken from a vessel 1. With the help of a pump 2 a sample can be provided with a suitable additive from a source 3. Should the sample be of blood, the additive can consist of heparin or another anti-coagulant. Should the sample be taken from, for example, a fermentation process, the additive can consist of an inhibitor,which interrupts all processes in the sample during its transport to the measuring unit. The additive is applied via a conduit 4. The sample, and any additives, is then fed through a conduit 5 via a valve 6 with a help of a pump 7 to a dialyzer 8. By means of the valve 6, a calibration solution from a source 9 thereof can alternatively be supplied instead. This is achieved via a conduit 9a.

In the dialyzer 8 the present substance is transferred to a carrier fluid or dialysis fluid from a source 10 thereof. This is achieved via a conduit 11 with a pump 12. The substance transferred with a help of dialysis is then fed via a conduit 13 through a valve 14 which either directs the flow through an enzyme column 15 or, via a conduit 16, bypasses the column and directs the flow directly to a conductivity meter 17. In this way the concentration of the compound can be measured after the effect of the enzyme column 15, whilst, at the same time, a 0-value can be obtained when the substance bypasses the enzyme column. The carrier fluid including the substance or broken down substance is then conducted via a conduit 18 to a waste collection point 19. The sample exposed to dialysis is also fed to the same waste collection point via a conduit 20. By means of the dashed lines there is shown how the valves and pumps of the system are controlled with a help of a processor unit 21 and a computer 22. These units are provided with the measured results obtained from the conductivity meter 17, which results can be shown on a display 23. Reference numeral 24 denotes a keyboard by means of which the shown system can be provided with a desired program.

Fig 2 shows the result of using the system according to the invention on a patient treated with hemodialysis. The X-axis represents the duration of dialysis expressed in minutes whilst the Y-axis represents the urea concentration in a patients blood expressed in millimoles. The black dots represent the results of off-line analysis performed in a conventional way. The analysis was carried out in such a way that blood from a patient, which was subjected to hemodialysis, was dialyzed with the help of a system substantially the same as that shown in fig 1. The source 3 consisted of heparin as anticoagulant. The dialysis was of course performed with normal dialysis fluid. In the system shown in fig 1, however, a water solution of glycine prepared with pure water of 50 mM concentration, and adjusted to pH 8.8 with sodium hydroxide, was used instead. Urea was transferred to this very low conductivity solution by dialysis in the dialyzer 8 so that it then was converted in the enzyme column 15 by the enzyme urease. As is evident from fig 2, a good correlation exists between the values thus obtained and the values obtained by conventional urea analysis.

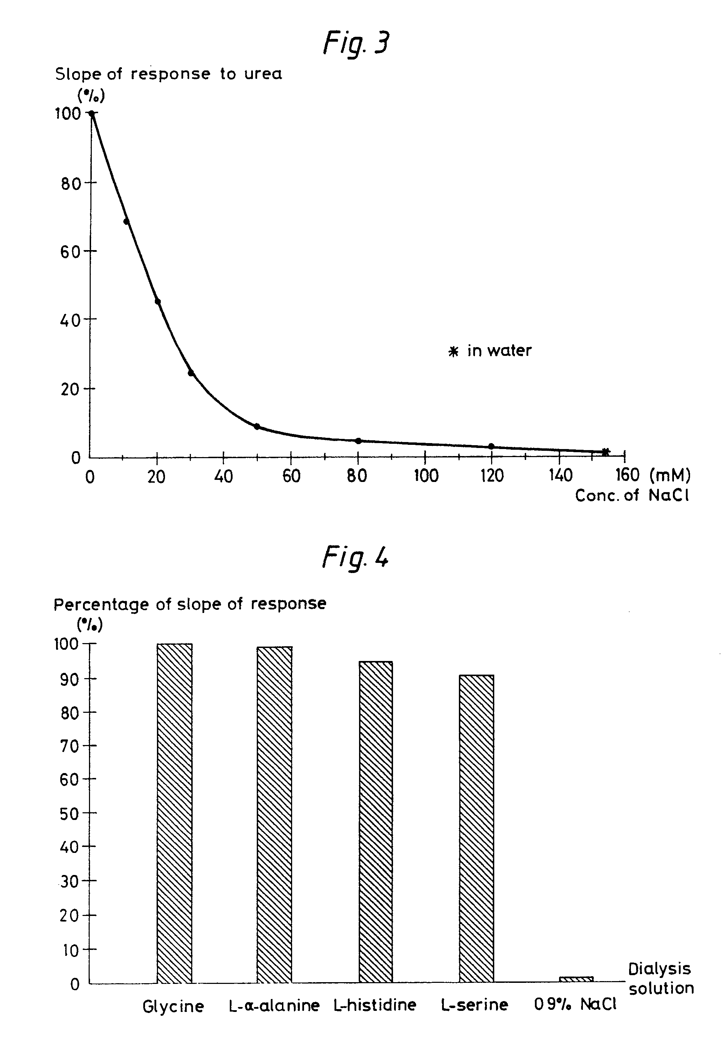

To investigate the influence of the carrier fluids own conductivity, a test was made using various carrier fluids, each consisting of a solution of 50 mM glycine in pure water adjusted to pH 8.8. This adjustment was achieved using sodium hydroxide. Various concentrations of sodium chloride where added to this carrier fluid. The result is shown in fig 3. The X-axis represents the concentration of sodium chloride expressed in millimoles whilst the Y-axis represents the percentage of slope of response to urea. From the results it is evident that the carrier fluid preferably should not contain any sodium chloride or other electrically conducting compounds.

To show that compounds other than glycine can be used in a method according to the invention, comparison tests with the compounds indicated in fig 4 where performed. The result shows that using L-alpha-alanine, L-histidine respectively L-serine the graph can be compared with that of fig 2 with the only difference being that the slope fluctuates somewhat. In each case when using the three above-mentioned compounds, the same solution as for the experiment with glycine was used, that is distilled water with 50 mM concentration adjusted to pH 8.8 using sodium hydroxide.

For comparison purposes, on the very right of fig 4 the result of an experiment using a solution of 0.9 % sodium chloride in distilled water is shown. Such a solution can obviously not be used for carrying out the method according to the invention. Instead, a carrier fluid should be used which is electrically non-conducting or very slightly conducting. The choice of such a fluid is however not restricted to just the above-mentioned solutions.

Naturally, the invention is not limited simply to that which is described above, but may be varied within the scope of the following claims. For example, clearly no dialysis is required if the tested compound is already present in a non-conducting or very slightly conducting solution. Furthermore the invention can of course be applied to many other compounds other than those mentioned. The only requirement is that the compound, from the outset, is non-conducting or very slightly conducting and can occur in a solution or in the form a dispersion in a non-conducting or very slightly conducting solution or can be transferred to such a solution with the help of, for example, dialysis.

## Claims

1. System for measuring the concentration of a substance in a solution or dispersion thereof, whereby the substance consists of a compound which is non-electrically conducting or very slightly conducting, characterized by means (15) for converting the substance in such a way that electrically conducting ions appear, and by means (17) for measuring these ions which are proportional to said degree of concentration.

2. System according to claim 1, characterized in that said means for converting the substance consists of an enzyme column (15).

3. System according to claim 1 or 2, characterized in that said means for measuring the appearing ions consists of a conductivity meter (17).

4. System according to anyone of the previous claims, whereby said substance occurs in an electrically conducting solution, characterized by means (8) for transferring the substance in its original form, or after converting appearing ions, to an electrically non-conducting or very slightly conducting solution before the measuring of the ions.

5. System according to claim 4, characterized in that said means for transferring the substance consists of a dialyzer, a filter or similar (8), whereby the transferring occurs by diffusion and/or filtration.

6. Method for measuring the concentration of a substance in a solution or dispersion thereof, whereby the substance consists of a compound which is non-electrically conducting or very slightly conducting, characterized in that the compound is converted in such a way that electrically conducting ions appear and that these ions are measured, the ions being proportional to the degree of concentration of the substance.

7. Method according to claim 6, characterized in that the conversion occurs with a help of an enzyme column (15) and the measuring of the appeared ions with a help of a conductivity meter (17).

8. Method according to either of claims 6 and 7, whereby said substance occurs in an electrically conducting solution, characterized in that this is first transferred to a non-electrically conducting or very slightly conducting solution before the measuring occurs.

9. Method according to claim 8, characterized in that both the conversion and the measuring occur after the transferring.

10. Method according to anyone of claims 6-9, characterized in that the measuring is carried out in a water solution which is prepared with, for example, distilled water.

**11.** Method according to anyone of claims 6-10, characterized in that the measuring is carried in a solution of low or non-conducting aminoacide.

**12.** Method according to claim 11, characterized in that the measuring occurs in a solution of anyone of the following aminoacides; L and/or D-alanine, L and/or D-histidine, L and/or D-serine or glycine, preferably glycine.

**13.** Method according to anyone of claims 6-12, characterized in that the substance to be measured consists of urea, for example in blood, whereby the conversion preferably occurs with the help of the enzyme urease.

**14.** Method according to anyone of claims 7-12, characterized in that an enzyme is used which is active on carbon-nitrogen bonds for measuring the present substance in anyone of the following combinations;
Asparaginase - L-Asparagine
Glutaminase - L-Glutamine
Penicillin amidase - Penicillin
Aminoacylase - N-acyl-amino acid
Citrullinase - L-Citrulline
Glutamin-(asparagin-)ase - L-Glutamine
preferably Urease - Urea.

## Fig. 1

## Fig. 2

### Urea concentrations during patient dialysis treatment

$y = 21.481 * \exp(-0.00363x) \quad r = 0.964$

## Fig. 3

Slope of response to urea
(%)

\* in water

Conc. of NaCl

## Fig. 4

Percentage of slope of response
(%)

Glycine    L-α-alanine    L-histidine    L-serine    0 9% NaCl

Dialysis
solution